(19) Europäisches Patentamt
European Patent Office
Office européen des brevets

(11) Publication number: **0 012 071 A1**

(12) **EUROPEAN PATENT APPLICATION**

(21) Application number: 79400904.3

(22) Date of filing: 22.11.79

(51) Int. Cl.³: C 07 D 471/10
C 07 D 487/10, A 61 K 31/445
A 61 K 31/40
//C07D207/12, (C07D471/10, 221/00, 209/00), (C07D487/10, 209/00, 209/00)

(30) Priority: 24.11.78 US 963258

(43) Date of publication of application: 11.06.80 Bulletin 80/12

(84) Designated Contracting States: AT BE CH DE FR GB IT LU NL SE

(71) Applicant: SYNTEX (U.S.A.) INC.
3401 Hillview Avenue
Palo Alto, California 94304(US)

(72) Inventor: Pfister, Jurg R.
1500 Oak Avenue
Los Altos, California 94022(US)

(74) Representative: Rinuy, Guy et al,
Cabinet Rinuy et Santarelli 14, Avenue de la Grande Armée
F-75017 Paris(FR)

(54) **Azoniaspiro derivatives, process for their preparation and pharmaceutical compositions containing them.**

(57) Compounds of the formula

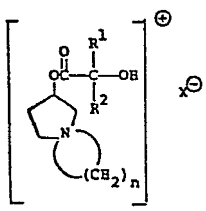

(I)

wherein $R^1$ and $R^2$ are the same or different and are $C_1$ to $C_6$ alkyl; $C_5$ or $C_6$ cycloalkyl; $C_5$ or $C_6$ cycloalkenyl; phenyl, optionally substituted with a substituent selected from the group $C_1$ to $C_6$ alkyl, $C_1$ to $C_6$ alkoxy and halo; or $C_4$ or $C_5$ heterocyclic aryl, the heteroatom selected from the group oxygen, nitrogen and sulfur;

n is the integer 4 or 5; and

X is a pharmaceutically acceptable anion.

These compounds are useful as anticholinergic agents.

The compounds of Formula (I) are prepared by reacting a compound represented by formula

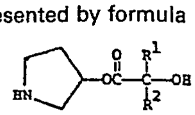

with a compound of the formula $X(CH_2)_nX$, wherein $R^1$, $R^2$, X and n are defined above.

EP 0 012 071 A1

Croydon Printing Company Ltd.

0012071

TITLE MODIFIED
see front page



ANTICHOLINERGIC BRONCHODILATORS

This invention relates to glycolic acid esters and pharmaceutically acceptable, non-toxic salts thereof and to methods for preparing these compounds. More particularly this invention relates to azoniaspiro glycolates, and to pharmaceutical compositions comprising one or more of the above compounds and to methods for achieving anticholinergic effects in mammals by using these compounds.

In summary, the compounds in accordance with the present invention are represented by the formula

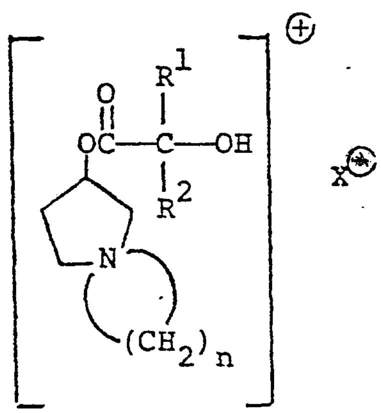

(I)

wherein $R^1$ and $R^2$ are the same or different and are $C_1$ to $C_6$ alkyl; $C_5$ or $C_6$ cycloalkyl; $C_5$ or $C_6$ cycloalkenyl; phenyl optionally substituted with $C_1$ to $C_6$ alkyl, $C_1$ to $C_6$ alkoxy or halo; or $C_4$ or $C_5$ heterocyclic aryl,

the heteroatom being oxygen, nitrogen or sulfur;

n is the integer 4 or 5; and

X is a pharmaceutically acceptable anion.

The process for preparing the compounds of the present invention of formula I comprises treating a compound of the formula

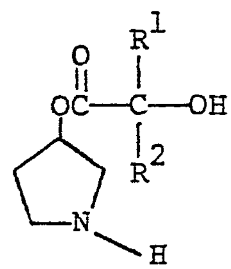

with $X(CH_2)_nX$, where $R^1$, $R^2$, X and n are as defined above, optionally with heat and a catalyst.

The compounds are useful as anticholinergic bronchodilators. A pharmaceutical composition of the present invention includes usual pharmaceutically acceptable forms such as solids, powders, solutions, suspensions, etc. and comprises one or more of the compounds of formula I in combination with a suitable pharmaceutical solvent or dispersant, i.e., sterile water or pharmaceutical solid excipients.

In the compounds of the present invention of formula I, $R^1$ and $R^2$ are the same or different and are preferably $C_1$ to $C_4$ alkyl, cyclopentyl, cyclohexyl, cyclopent-2-enyl, cyclohex-3-enyl, phenyl optionally substituted with a substituent selected from the group $C_1$ to $C_4$ alkyl, $C_1$ to $C_4$ alkoxy, chloro and bromo, or thienyl and X is selected from the group methanesulfonate, benzenesulfonate, p-toluenesulfonate, chloride, bromide and iodide. More preferably X is selected from the group methanesulfonate, benzenesulfonate, chloride and bromide.

Most preferred compounds of formula II are those

where $R^1$ and $R^2$ are the same or different and are cyclopentyl, cyclohexyl or phenyl and X is chloride or bromide.

Particularly preferred compounds of formula II are:

2-(2,2-diphenyl-2-hydroxyacetoxy)-5-azoniaspiro[4.4]nonane chloride ;

2-(2,2-diphenyl-2-hydroxyacetoxy)-5-azoniaspiro[4.5]decane chloride ;

2-(2-cyclohexyl-2-hydroxy-2-phenylacetoxy)-5-azoniaspiro[4.4]nonane chloride;

2-(2-cyclopentyl-2-hydroxy-2-phenylacetoxy)-5-azoniaspiro[4.4]nonane chloride; and

2-(2-cyclopentyl-2-hydroxy-2-phenylacetoxy)-5-azoniaspiro[4.4]nonane bromide.

The compounds of the present invention of Formula (I) are made by the following process depicted schematically:

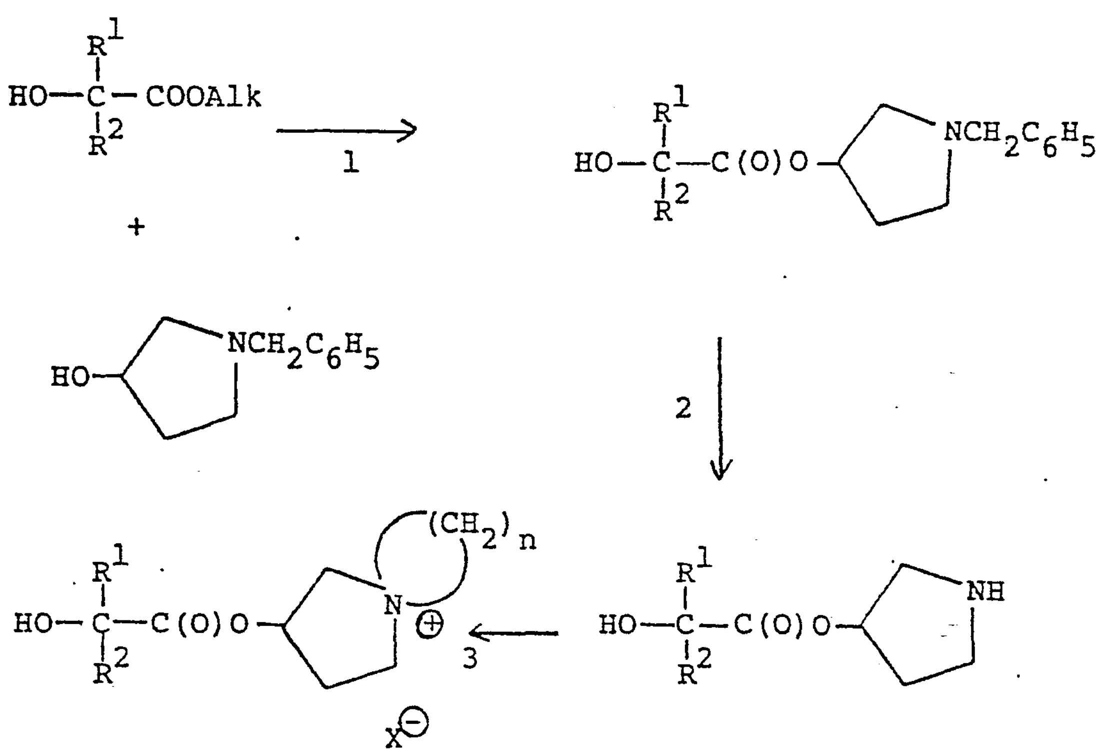


I

The initial reaction, as shown in reaction step 1

involves the transesterification of 1-benzyl-3-pyrrolidinol with an alkyl ester of benzilic acid. As depicted in this step, ALK can be any lower alkyl group including methyl, ethyl, i-propyl, and the like. While the benzyl pyrrolidinol compound is the preferred starting material in this step, any of the prior art groups generally known as protecting groups can be employed as the substituent at the nitrogen atom. These include, for example, the groups diphenylmethyl, o-nitrobenzyl, p-nitrobenzyl, 3,5-dinitrobenzyl, p-methoxybenzyl, pivaloyloxymethyl, trichloroethyloxycarbonyl and the like. The transesterification is conducted in the presence of strong base, for example an alkali metal hydride, optionally in the presence of inert organic solvent, at times and temperatures sufficient to complete the reaction, typically 30 minutes to 24 hours at 50° to 150°, preferably 4 hours under reflux conditions, e.g., 100°. Solvents for this reaction include the aliphatic hydrocarbon solvents, such as hexane, heptane, octane, etc.; and the aromatic solvents such as benzene, toluene, etc.

The glycolic acid ester, preferably isolated from step 1 is subjected to hydrogenolysis to remove the protecting group on the nitrogen atom. Preferably the reaction is conducted in aqueous acid solutions, most preferably at a pH of about 4 in the presence of a hydrogenolysis catalyst for a time sufficient to assure completeness of the reaction. Various metal catalysts known in the art can be employed in this step including platinum, Raney nickel, etc. Typically the catalyst is palladium optionally on a substrate, e.g., carbon, aluminum oxide, barium sulfate, etc. The catalyst is most preferably palladium oxide or palladium black. The reaction is conducted at about room temperature and terminated when complete, i.e., after hydrogen uptake has ceased.

Formation of the spiro compounds of Formula (I) can be accomplished by treatment of the reaction intermediate of step 2 with a compound of the formula $X(CH_2)_nX$ (wherein n and X are set forth above), i.e. an alkylene dihalide such as 1,4-dibromobutane, 1,4-dichlorobutane, 1,5-dichloropentane and the like or an alkylene ditosylate or dimesylate in a suitable inert organic solvent in the presence of a base. Preferably the alkylene dihalides are employed in reaction step 3, most preferably the alkylene dibromides with the base being preferably an organic base such as a trialkylamine, e.g. triethylamine, or a heteroalicyclic amine, e.g. N-methylmorpholine, quinuclidine, etc. The reaction solvent is typically an inert polar organic solvent such as acetonitrile, dimethylsulfoxide, dimethylformamide, and the like, the reaction being conducted for a time and at a temperature sufficient to assure completeness of reaction, typically 75°-150°, for 24 to 72 hours.

The compounds of the invention are predominantly antagonists of acetyl choline and are particularly effective as bronchodilators. As such, the compounds of this invention are typically administered in dosages of about from 0.01 to 5 mg per kg. of body weight. The precise effective dosage will, of course, vary depending upon the mode of administration, the condition being treated, and the host. Where the compounds are used as pulmonary anticholinergics in mammals they are typically administered either orally, intravenously, or by inhalation.

The compounds of the present invention can be administered in a wide variety of dosage forms, either alone or in combination with other pharmaceutically compatible medicaments, in the form of pharmaceutical compositions suited for oral, parenteral or aerosol administration. The compounds are typically administered

as pharmaceutical compositions consisting essentially of the compounds of the invention and a pharmaceutical carrier. The pharmaceutical carriers can be either a solid material or a liquid in which the compound is dissolved, dispersed or suspended, and can optionally contain small amounts of preservatives and/or pH-buffering agents. Suitable preservatives which can be used include, for example, benzyl alcohol and the like. Suitable buffering agents include, for example, pharmaceutical phosphate salts and the like.

The liquid compositions can, for example, be in the form of solutions, emulsions, suspensions, syrups, or elixirs and optionally can contain small quantities of preservatives and/or buffering agents, and preferably contain the therapeutic agents in convenient unit dosage.

The solid compositions can take the form of tablets, powders, capsules, pills and the like, preferably in unit dosage forms for simple administration or precise dosages. Suitable solid carriers include, for example, pharmaceutical grades of starch, lactose, sodium saccharine, sodium bisulfite and the like.

Also based on studies on related compounds, it can be predicted that a number of the present compounds will exhibit useful anticholinergic activity when administered topically, intradermally, or subcutaneously.

The compounds of formula I can be administered as racemic mixtures or they can be administered as resolved enantiomers or optical isomers. In some instances, one enantiomer or optical isomer exhibits a greater anticholinergic effect than does the other corresponding enantiomer or optical isomer.

As used in the specification and the appended claims, the following terms have the meaning indicated.

The term pharmaceutically acceptable anion refers to the negatively charged ions which do not adversely effect

the pharmacological activity of the compound, that is, they are essentially non-toxic at the level used and the anticholinergic bronchodilating activity is not negated. Suitable anions include, e.g. methanesulfonate, benzenesulfonate, p-toluenesulfonate, nitrate, chloride, bromide, iodide and the like.

The term "$C_1$ to $C_6$ alkyl" refers to a straight or branched chain, monovalent substituent consisting solely of carbon and hydrogen, containing no unsaturation, and having from 1 to 6 carbon atoms. Examples of such alkyl groups are methyl, ethyl, n-propyl, i-propyl, n-butyl, t-butyl, n-hexyl, 2-methylpentyl and the like.

The term "$C_1$ to $C_6$ alkoxy" refers to the above disclosed alkyl groups linked through an ether linkage, having the free valence from the ether oxygen. Examples of such groups include methoxy, ethoxy, n-propoxy, i-propoxy, n-butoxy, t-butoxy, n-hexyloxy, and the like.

The term " $C_5$ to $C_6$ cycloalkyl" refers to a 5 or 6 membered monovalent ring containing only hydrogen and carbon that is fully saturated such as exemplified by cyclopentyl or cyclohexyl. "$C_5$ to $C_6$ cycloalkenyl" differs from the above cycloalkyl by having in the ring at least one unsaturated site. Such includes 1-, 2- or 3-cyclopentenyl, 1-, 2- or 3-cyclohexenyl, 1,4-cyclohexadienyl, 1,3-cyclopentadienyl, and the like.

The term "halide" refers to fluoride, chloride, bromide and iodide.

The term "heterocyclic aryl, the heteroatom selected from oxygen, nitrogen and sulfur" is intended to mean the monovalent heterocyclic radicals of aromatic character containing , in addition to the heteroatom, 4 or 5 carbon atoms in the ring. Examples of these radicals are pyrryl, for example 2- or 3-pyrryl, pyridyl, for example 2-, 3- or 4-pyridyl, thienyl, for example 2- or 3-thienyl and furyl, for example 2-furyl or 3-furyl.

The term "phenyl optionally substituted with a substituent selected from the group $C_1$ to $C_6$ alkyl, $C_1$ to $C_6$ alkoxy and halo" is intended to include unsubstituted phenyl, monosubstituted phenyl and polysubstituted phenyl. Such include methylphenyl, for example 2- or 3-methylphenyl, dimethylphenyl, for example 2,4- or 3,5-dimethylphenyl, methoxyphenyl, for example 2- or 3-methoxyphenyl, dimethoxyphenyl for example 2,4- or 3,5-dimethoxyphenyl, halophenyl for example 4-chlorophenyl, or 4-bromophenyl, or dihalophenyl, for example 2,4-dichlorophenyl or 2,4-dibromophenyl.

The compounds of formula I may possess a chiral center. Accordingly, the compounds of the invention may be prepared in either their optically active form or as a racemic mixture. Unless otherwise specified, the compounds described herein are all in the racemic form. However, the scope of the subject invention is not to be considered limited to the racemic form, but to encompass the individual optical isomers of the compounds of the present invention.

Where desired the individual diastereomeric and optically isomeric compounds can be isolated by conventional separation and purification procedures in the case of diastereomers and by conventional resolution procedures in the case of optical isomers. Optimum physical, or physical-chemical, separation procedures and resolution procedures can be obtained by routine trial and error procedures well within the scope of those skilled in the art.

A further understanding of the invention can be had from the following non-limiting Preparations and Examples. As used hereinabove and below unless expressly stated to the contrary, all temperatures and temperature ranges refer to the Centigrade system and the terms ambient or room temperature refer to about 20°C. The

term percent or (%) refers to weight percent and the term mole and moles refers to gram moles. The term equivalent refers to a quantity of reagent equal in moles to the moles of the preceding or succeeding reactant recited in that Preparation or Example in the terms of moles of finite weight or volume. As noted earlier, compounds having asymmetric centers and optical activity are isolated in their racemic form ($\pm$) unless otherwise indicated.

PREPARATION 1

To a solution of 12g methyl benzilate and 8.2g 1-benzyl-3-pyrrolidinol in 200ml n-heptane is added 100mg sodium hydride and the mixture refluxed for 4 hours, separating the methanol formed in a Dean-Stark separator. The cooled organic phase is washed with water and then extracted with 2N HCl. The aqueous phase is made alkaline with solid $K_2CO_3$ and extracted with ether to give 1-benzyl-3-(2,2-diphenyl-2-hydroxyacetoxy)-pyrrolidine as an oil (18.2g).

In a similar manner, using the following compounds in place of methyl benzilate:

methyl dimethylglycolate;
methyl diethylglycolate;
methyl di-n-butylglycolate;
methyl methyl-n-butylglycolate;
ethyl methylcyclopentylglycolate;
ethyl dicyclopentylglycolate;
ethyl methylcyclopent-2-enylglycolate;
ethyl methylpentylglycolate;
methyl methyl(3-methylphenyl)glycolate;
methyl methyl(4-chlorophenyl)glycolate;
methyl methylpyrrol-3-ylglycolate;
methyl methylfur-2-ylglycolate;
methyl methylthien-2-ylglycolate;

methyl cyclohexylphenylglycolate;
methyl cyclopentylphenylglycolate;
methyl dicyclohexylglycolate; and
methyl isopropylphenylglycolate
are prepared the following compounds:

1-benzyl-3-(2,2-dimethyl-2-hydroxyacetoxy)pyrrolidine;

1-benzyl-3-(2,2-diethyl-2-hydroxyacetoxy)pyrrolidine;

1-benzyl-3-(2,2-di-n-butyl-2-hydroxyacetoxy)pyrrolidine;

1-benzyl-3-(2-methyl-2-n-butyl-2-hydroxyacetoxy)-pyrrolidine;

1-benzyl-3-(2-methyl-2-cyclopentyl-2-hydroxyacetoxy)pyrrolidine;

1-benzyl-3-(2,2-dicyclopentyl)-2-hydroxyacetoxy)-pyrrolidine;

1-benzyl-3-[2-methyl-2-(cyclopent-2-enyl)-2-hydroxy-acetoxy]pyrrolidine;

1-benzyl-3-(2-methyl-2-phenyl-2-hydroxyacetoxy)pyrrolidine;

1-benzyl-3-[2-methyl-2-(3-methylphenyl)-2-hydroxy-acetoxy]pyrrolidine;

1-benzyl-3-[2-methyl-2-(4-chlorophenyl)-2-hydroxy-acetoxy]pyrrolidine;

1-benzyl-3-[2-methyl-2-(pyrrol-3-yl)-2-hydroxy-acetoxy]pyrrolidine;

1-benzyl-3-[2-methyl-2-(fur-2-yl)-2-hydroxy-acetoxy]pyrrolidine; and

1-benzyl-3-[2-methyl-2-(thien-2-yl)-2-hydroxy-acetoxy]pyrrolidine;

1-benzyl-3-(2-cyclohexyl-2-phenyl-2-hydroxyacetoxy)-pyrrolidine;

1-benzyl-3-(2-cyclopentyl-2-phenyl-2-hydroxyacetoxy)-pyrrolidine;

2-(2,2-dimethyl-2-hydroxyacetoxy)-5-azoniaspiro[4.4]nonane chloride;

2-(2,2-diethyl-2-hydroxyacetoxy)-5-azoniaspiro[4.4]nonane chloride;

2-(2,2-di-n-butyl-2-hydroxyacetoxy)-5-azoniaspiro[4.4]nonane chloride;

2-(2-methyl-2-n-butyl-2-hydroxyacetoxy)-5-azoniaspiro[4.4]nonane chloride;

2-(2-methyl-2-cyclopentyl-2-hydroxyacetoxy)-5-azoniaspiro[4.4]nonane chloride;

2-(2,2-dicyclopentyl-2-hydroxyacetoxy)-5-azoniaspiro[4.4]nonane chloride;

2-[2-methyl-2-(cyclopent-2'-enyl)-2-hydroxyacetoxy]-5-azoniaspiro[4.4]nonane chloride;

2-(2-methyl-2-phenyl-2-hydroxyacetoxy)-5-azoniaspiro[4.4]nonane chloride;

2-[2-methyl-2-(3-methylphenyl)-2-hydroxyacetoxy]-5-azoniaspiro[4.4]nonane chloride;

2-[2-methyl-2-(3-hexylphenyl)-2-hydroxyacetoxy]-5-azoniaspiro[4.4]nonane bromide

2-[2-methyl-2-(4-chlorophenyl)-2-hydroxyacetoxy]-5-azoniaspiro[4.4]nonane chloride;

2-[2-methyl-2-(pyrrol-3-yl)-2-hydroxyacetoxy]-5-azoniaspiro[4.4]nonane chloride;

2-[2-methyl-2-(fur-2-yl)-2-hydroxyacetoxy]-5-azoniaspiro[4.4]nonane chloride;

2-[2-methyl-2-(thien-2-yl)-2-hydroxyacetoxy]-5-azoniaspiro[4.4]nonane chloride;

2-[2.2-dicyclohexyl-2-hydroxyacetoxy)-5-azoniaspiro[4.4]nonane bromide, m.p. 246-248°C; and

2-(2-isopropyl-2-phenyl-2-hydroxyacetoxy)-5-azoniaspiro[4.4]nonane bromide.

EXAMPLE 2

A. By following in principle the procedure of Example 1, but substituting 1,5-dibromopentane for

1-benzyl-3-(2.2-dicyclohexyl-2-hydroxyacetoxy)pyrrolidine; and

1-benzyl-3-(2-isopropyl-2-phenyl-2-hydroxyacetoxy)-pyrrolidine.

PREPARATION 2

A. 18.2g of the amino ester of Preparation 1 is dissolved in 900ml ethanol, and sufficient ethanolic hydrochloric acid is added to obtain a pH of about 4. The resulting solution is hydrogenated over 2.2g 10% palladium on carbon at normal pressure and room temperature. After hydrogen uptake has ceased (about 1.2 l), the solution is filtered through Celite and the filtrate concentrated to about 100ml on a rotary evaporator. The residue is diluted with 500ml water, made alkaline with solid potassium carbonate, and extracted with ether to give 12.4g of an oil which when crystallized from ether/hexane yields 3-(2,2-diphenyl-2-hydroxyacetoxy)pyrrolidine, mp. 123-124°.

B. In a similar manner, the benzylpyrrolidines of Preparation I are converted into their respective pyrrolidines.

EXAMPLE 1

A. A solution of 12.4g of the pyrrolidine of Preparation 2, Part A, 11g of 1,4-dichlorobutane and 8ml of triethylamine in 180ml acetonitrile is refluxed for 48 hours. The cooled reaction mixture is evaporated to dryness, the residue dissolved in 350ml water and extracted with ether. The aqueous phase is extracted continuously with ethyl acetate for 48 hours to give 8.3g of 2-(2,2-diphenyl-2-hydroxyacetoxy)-5-azoniaspiro-[4.4]nonane chloride, mp 150-152° (from MeOH/AcOEt).

B. In a similar manner the pyrrolidines illustrated in Preparation 2 are converted into the following compounds:

2-[2-isopropyl-2-phenyl-2-hydroxyacetoxy)-5-azoniaspiro[4.5]decane bromide.

EXAMPLE 3

To a solution of methyl 2-cyclohexyl-2-phenylglycolate (3.55g) and 1-benzylpyrrolidin-3-ol (2.3g) in n-heptane (100ml) is added 50 mg of sodium hydride. The resulting mixture refluxed for 9 hours through molecular sieves (type 4A). The cooled reaction mixture is diluted with ether (200ml) and washed successively with water and dilute hydrochloric acid. The acidic extract is made basic by the addition of solid potassium carbonate and extracted with ether to give 3-(2-cyclohexyl-2-hydroxy-2-phenylacetoxy)-1-benzylpyrrolidine (2.7g) as a viscous oil.

To a solution of 3-(2-cyclohexyl-2-hydroxy-2-phenylacetoxy)-1-benzylpyrrolidine (2.7g) in 80ml of ethanol is added enough ethanolic hydrogen chloride to obtain a pH of 4. The resulting solution is hydrogenated over 250mg of 10% Pd-C (uptake 183ml $H_2$). The catalyst is filtered off and the filtrate evaporated. The residue is dissolved in water (100ml) and extracted with ether. The aqueous phase is made basic with solid potassium carbonate and extracted with ether to give 3-(2-cyclohexyl-2-hydroxy-2-phenylacetoxy)-pyrrolidine (1.8g) as a viscous oil.

A solution of 3-(2-cyclohexyl-2-hydroxy-2-phenylacetoxy)pyrrolidine (1.8g) and 1.4-dibromobutane (2.7g) is refluxed for 3 hours. Triethylamine (1.15ml) is then added, and refluxing is continued for 9 hours. The reaction mixture is evaporated to dryness and the residue distributed between water (50ml) and ether (50ml). The aqueous phase is extracted with ethyl acetate in a continuous extraction apparatus to give 1.1g of 2-(2-cyclohexyl-2-hydroxy-2-phenylacetoxy)-5-azoniaspiro[4.4]nonane bromide as a white foam having the

1,4-dichlorobutane one obtains

2-(2,2-diphenyl-2-hydroxyacetoxy)-5-azoniaspiro[4.5]decane chloride, mp 237-238°.

B. In a similar manner, the pyrrolidines illustrated in Preparation 2 are converted into the following compounds:

2-(2,2-dimethyl-2-hydroxyacetoxy)-5-azoniaspiro[4.5]decane bromide;

2-(2,2-diethyl-2-hydroxyacetoxy)-5-azoniaspiro[4.5]decane bromide;

2-(2,2-di-n-butyl-2-hydroxyacetoxy)-5-azoniaspiro[4.5]decane bromide;

2-(2-methyl-2-n-butyl-2-hydroxyacetoxy)-5-azoniaspiro[4.5]decane bromide;

2-(2-methyl-2-cyclopentyl-2-hydroxyacetoxy)-5-azoniaspiro[4.5]decane bromide;

2-(2,2-dicyclopentyl-2-hydroxyacetoxy)-5-azoniaspiro[4.5]decane bromide;

2-[2-methyl-2-(cyclopent-2'-enyl)-2-hydroxyacetoxy]-5-azoniaspiro[4.5]decane bromide;

2-(2-methyl-2-phenyl-2-hydroxyacetoxy)-5-azoniaspiro[4.5]decane bromide;

2-[2-methyl-2-(3-methylphenyl)-2-hydroxyacetoxy]-5-azoniaspiro[4.5]decane bromide;

2-[2-methyl-2-(4-chlorophenyl)-2-hydroxyacetoxy]-5-azoniaspiro[4.5]decane bromide;

2-]-methyl-2-(pyrrol-3-yl)-2-hydroxyacetoxy]-5-azoniaspiro[4.5]decane bromide;

2-[2-methyl-2-(fur-2-yl)-2-hydroxyacetoxy]-5-azoniaspiro[4.5]decane bromide;

2-[2-methyl-2-(thien-2-yl)-2-hydroxyacetoxy]-5-azoniaspiro[4.5]decane bromide;

2-[2,2-dicyclohexyl-2-hydroxyacetoxy)-5-azoniaspiro[4.5]decane bromide; and

following physical characteristics:

Mass spectrum: M/e = 236,205,203,189,152,105,82,77,55

NMR: δ = 1.08(m), 1.5(m), 2.07(m), 3.58(m), 5.42(m), 5.73(s), 7.45(m)ppm.

B. In a similar manner, but substituting methyl 2-cyclopentyl-2-phenylglycolate for 2-cyclohexyl-2-phenylglycolate, one obtains 2-(2-cyclopentyl-2-hydroxy-2-phenylacetoxy)-5-azoniaspiro[4.4]nonane bromide having the NMR characteristics:

NMR: δ = 1.43(m), 2.03(m), 3.55(m), 5.40(m), 5.87(s), 7.42(m) ppm.

C. By following in principle the procedure of Parts A and B of this example but substituting 1,5-dibromopentane for 1,4-dibromobutane, one obtains

2-(2-cyclohexyl-2-phenyl-2-hydroxyacetoxy)-5-azoniaspiro[4.5]decane bromide and

2-(2-cyclopentyl-2-phenyl-2-hydroxyacetoxy)-5-azoniaspiro[4.5]decane bromide.

## EXAMPLE 4 - $LD_{50}$ VALUES

The intraperitoneal $LD_{50}$ values in male mice (ICR-Simonsen) were determined, using 5 mice at each dosage level, for the compounds of this invention listed below and represented by Formula (I). The mice were observed for mortality for six hours after dosing. The $LD_{50}$ values were calculated using the Litchfield-Wilcoxon method as described by Litchfield, J.T., Jr. and Wilcoxon, S. in Journal of Pharmacology and Experimental Therapeutics, Vol. 96, p.99, 1949.

| Compound (See Formula I) | Dosage Level (mg/kg) | $LD_{50}$ (95% Confidence Limits) mg/kg |
|---|---|---|
| 1. $R^1$ = phenyl, $R^2$ = cyclohexyl n = 4, X = Br | 80, 60, 50, 40 | 54.5(47.8-62.1) |
| 2. $R^1$ = phenyl, $R^2$ = cyclopentyl, n = 4, X = Br | 80, 60, 40 | 67.5(59.7-76.3) |
| 3. $R^1$= $R^2$ = phenyl n = 4, X = Br | 80, 60, 50, 40 | 47(40.8-54.1) |

## EXAMPLE 5 - Biological Data

Guinea Pigs of either sex were anesthetized with pentobarbital sodium intraparitoneally at a dose of 30-40mg/kg. The animals were ventilated mechanically with room air via a tracheal cannula connected to a Harvard pump set at a volume of 10ml/kg body weight and at a frequency of 60 strokes/min. The right jugular vein was cannulated for intravenous drug administration and the left carotid artery was cannulated for blood pressure measurement. Inflation pressure was measured with a differential pressure transducer by connecting the high side of the gauge to an extension of the tracheal cannula and exposing the low side to atmospheric pressure.

Bronchoconstriction was elicited by I.V. injections of methacholine (2-8μg/kg). For each animal, a dose of bronchoconstrictor was selected which produced a moderate but easily detectable increase in tracheal inflation pressure with a return to baseline within five minutes. Each experiment consisted of a control period during which two successive and approximately equal bronchoconstriction responses were obtained.

Test compounds were then administered intraveneously with a challenge of bronchoconstricting agent 30 seconds after each compound dose and then at the following time intervals: 20, 30, 60, 90, 130, 150, and 180 minutes.

The results are set forth in Table I

TABLE 1

| Compound | No. of Test Animals | Onset of Action | Duration (t 1/2) | Duration Relative to IB | Peak $ED_{50}$ | Potency Relative to IB[b] |
|---|---|---|---|---|---|---|
| IB[b] | 7 | 10 min. | 48 min. | 1.0 | 2.0µg/kg | 1 |
| 1.[a] | 5 | 20 min. | 233 min. | 4.8 | 3.5µg/kg | 0.57 |
| 2.[a] | 4 | 5 min. | 96 min. | 2.0 | 1.3µg/kg | 1.54 |
| 3.[a] | 3 | 5 min. | 37 min. | 0.8 | 1.2µg/kg | 1.67 |

[a] See Example 4

[b] Ipratropium bromide

While the present invention has been described with reference to specific embodiments thereof, it should be understood by those skilled in the art that various changes may be made and equivalents may be substituted without departing from the true spirit and scope of the invention. In addition, many modifications can be made to adapt a particular situation, material or composition of matter, process, process step or steps or objective to the spirit of this invention without departing from its essential teachings.

In the claims:

1. A compound chosen from those represented by the formula

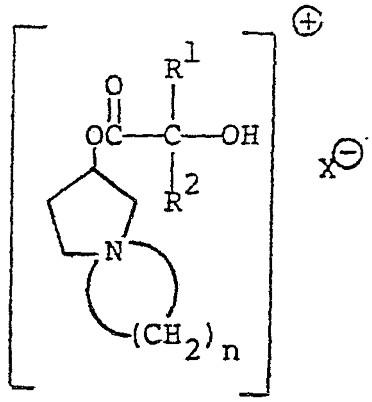

(I)

wherein $R^1$ and $R^2$ are the same or different and are $C_1$ to $C_6$ alkyl, $C_5$ or $C_6$ cycloalkyl, $C_5$ or $C_6$ cycloalkenyl, phenyl optionally substituted with a substituent selected from the group $C_1$ to $C_6$ alkyl, $C_1$ to $C_6$ alkoxy and halo, $C_4$ or $C_5$ heterocyclic aryl, the heteroatom being oxygen, nitrogen or sulfur;

n is the integer 4 or 5; and

X is a pharmaceutically acceptable anion.

2. A compound of Claim 1 wherein X is nitrate, methanesulfonate, benzenesulfonate, p-toluenesulfonate, chloride, bromide or iodide.

3. A compound of Claim 1 wherein $R^1$ and $R^2$ are the same or different and are cyclopentyl, cyclohexyl, cyclopent-2-enyl, cyclohex-3-enyl or phenyl optionally substituted with a substituent selected from the group $C_1$ to $C_4$ alkyl, $C_1$ to $C_4$ alkoxy, chloro and bromo and X is methanesulfonate, benzenesulfonate, chloride or bromide.

4. A compound of Claim 2 wherein $R^1$ and $R^2$ are the same or different and are cyclopentyl, cyclohexyl or phenyl and X is chloride or bromide.

5. A compound of Claim 4 wherein $R^1$ and $R^2$ are both phenyl and n is 4.

6. A compound of Claim 4 wherein $R^1$ is phenyl, $R^2$ is cyclohexyl and n is 4.

7. A compound of Claim 4 wherein $R^1$ is phenyl, $R^2$ is cyclopentyl and n is 4.

8. A compound of Claim 4 wherein $R^1$ and $R^2$ are both phenyl and n is 5.

9. A compound of Claim 4 wherein $R^1$ and $R^2$ are both cyclohexyl and n is 4.

10. A pharmaceutical composition which comprises a pharmaceutical carrier and an effective amount of at least one compound selected from the group recited in any of the preceding claims.

11. A pharmaceutical composition according to Claim 10, characterized in that it is an anti-cholinergic bronchodilating composition.

12. A method for effecting bronchodilation in mammals which comprises administering to said mammals a bronchodilating effective amount of at least one compound selected from the group recited in any of Claims 1 - 9.

13. A method for preparing a compound of Claim 1 which comprises reacting a compound represented by the formula

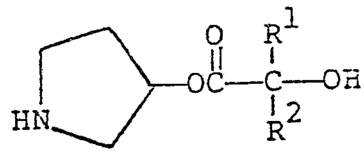

with a compound of the formula $X(CH_2)_nX$, wherein $R^1$, $R^2$, X and n are defined above.

European Patent Office

## PARTIAL EUROPEAN SEARCH REPORT

which under Rule 45 of the European Patent Convention shall be considered, for the purposes of subsequent proceedings, as the European search report

0012071

Application number

EP 79 40 0904

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| | FR - A - 1 439 086 (A.H. ROBINS)<br>* Claim 1 *<br>---- | 1 | C 07 D 471/10<br>487/10<br>A 61 K 31/445<br>31/40//<br>C 07 D 207/12<br>(C 07 D 471/10<br>221/00<br>209/00)<br>(C 07 D 487/10<br>209/00<br>209/00) |
| | | | TECHNICAL FIELDS SEARCHED (Int. Cl.³) |
| | | | C 07 D 471/10<br>487/10<br>A 61 K 31/445<br>31/40 |

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-11,13

Claims searched incompletely:

Claims not searched: 12

Reason for the limitation of the search: Method for treatment of the human or animal body by surgery or therapy (See article 52(4) of the European Patent Convention).

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| Place of search | Date of completion of the search | Examiner |
|---|---|---|
| The Hague | 22-02-1980 | ALFARO |